(19)

**Europäisches Patentamt**
**European Patent Office**
**Office européen des brevets**

(11)  **EP 4 351 412 B1**

(12)  # EUROPEAN PATENT SPECIFICATION

(45) Date of publication and mention
of the grant of the patent:
**01.10.2025  Bulletin 2025/40**

(21) Application number: **22773071.0**

(22) Date of filing: **29.08.2022**

(51) International Patent Classification (IPC):
***A61B 5/024*** *(2006.01)*     ***A61B 5/00*** *(2006.01)*
***A61B 5/1455*** *(2006.01)*

(52) Cooperative Patent Classification (CPC):
**A61B 5/681; A61B 5/14552; A61B 5/7203**

(86) International application number:
**PCT/US2022/041892**

(87) International publication number:
**WO 2023/028367 (02.03.2023 Gazette 2023/09)**

(54)  **ESTIMATING OXYGEN SATURATION USING GREEN OPTICAL LIGHT AS A FILTER**

SCHÄTZUNG DER SAUERSTOFFSÄTTIGUNG MIT GRÜNEM OPTISCHEM LICHT ALS FILTER

ESTIMATION DE LA SATURATION EN OXYGÈNE À L'AIDE D'UNE LUMIÈRE OPTIQUE VERTE EN TANT QUE FILTRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **27.08.2021   US 202163237976 P**

(43) Date of publication of application:
**17.04.2024   Bulletin 2024/16**

(73) Proprietor: **Google LLC
Mountain View, CA 94043 (US)**

(72) Inventors:
 • **KERNASOVSKIY, Sarah Ann Stokes
  Mountain View, California 94043 (US)**
 • **VAVADI, Hamed
  Mountain View, California 94043 (US)**

 • **RICHARDS, Peter
  Mountain View, California 94043 (US)**
 • **HENEGHAN, Conor Joseph
  Mountain View, California 94043 (US)**
 • **SUNDEN, Lindsey
  Mountain View, California 94043 (US)**
 • **SU, Hao-Wei
  Mountain View, California 94043 (US)**
 • **NIEHAUS, Logan Alexander
  Mountain View, California 94043 (US)**

(74) Representative: **Derry, Paul Stefan et al
Venner Shipley LLP
200 Aldersgate
London EC1A 4HD (GB)**

(56) References cited:
**WO-A1-2018/112401      US-A1- 2016 361 004
US-A1- 2020 253 562      US-B1- 10 568 525**

**EP 4 351 412 B1**

**Description**

FIELD

The present disclosure relates generally to measuring blood oxygen levels of a user.

BACKGROUND

User computing devices increasingly incorporate the ability to measure one or more metrics associated with human health or the human body. One such health-related metric is the amount of oxygen present in the blood of a user. However, a number of factors, including where on the body the measurement is taken can result in inaccurate measurements of oxygen levels in the blood when measured at particular parts of the human body. As such, improving the accuracy of such measurements can result in improved usefulness of wearable computing devices.

US2016/361004 discloses performing pulse oximetry commencing with receiving at least three light signals of three different wavelengths reflected from a human tissue. The human tissue includes a pulsatile tissue and a non-pulsatile tissue. Based on the three light signals, values of at least three functions are determined. The three functions are invariant to an oxygen saturation in the pulsatile tissue and depend on location of a sensor operable to detect the three light signals and pressure of the sensor on the human tissue. Based on the values of the three functions, non-pulsatile components are analyzed for intensities of a red light signal and infrared light signal reflected from the human tissue. The non-pulsated components are removed from the intensities to allow correct estimates of a ratio of the absorption coefficients, with the ratio being used to determine the oxygen saturation in the pulsatile tissue.

US 10568525 discloses creating a blood oxygen saturation (SpO2) value by receiving one or more photoplethysmography (PPG) signals for SpO2 detection from one or more PPG sensors; receiving one or more PPG signals for characterizing a heart rate from the one or more PPG sensors; using the one or more PPG signals for SpO2 detection, forming one or more SpO2 datasets wherein the SpO2 datasets respectively comprise one or more noise components; removing the one or more noise components from the one or more SpO2 datasets that are inconsistent with a feature of the one or more PPG signals characterizing the heart rate to produce one or more filtered SpO2 datasets; and using the one or more filtered SpO2 datasets, creating and storing the SpO2.

SUMMARY

Aspects and advantages of embodiments of the present disclosure will be set forth in part in the following description, or can be learned from the description, or can be learned through practice of the embodiments.

A first aspect provides a method for measuring blood oxygen saturation in a user as claimed in claim 1.

Another aspect provides a wearable computing device as claimed in claim 14.

Another aspect provides computer program instructions as claimed in claim 15.

Other aspects of the specification are directed to various systems, apparatuses, non-transitory computer-readable media, user interfaces, and electric devices.

These and other features, aspects, and advantages of various embodiments of the present disclosure will become better understood with reference to the following description and appended claims. The accompanying drawings, which are incorporated in and constitute a part of this specification, illustrate example embodiments of the present disclosure and, together with the description, serve to explain the related principles.

BRIEF DESCRIPTION OF THE DRAWINGS

Detailed discussion of embodiments directed to one of ordinary skill in the art are set forth in the specification, which refers to the appended figures, in which:

FIG. 1 illustrates an example of a front side of a wearable computing device in accordance with example embodiments of the present disclosure;
FIG. 2 illustrates an example of a rear side of a wearable computing device in accordance with example embodiments of the present disclosure;
FIG.3 illustrates an example graph representing an expected intensity of back-reflected light based on whether blood is oxygenated or deoxygenated;
FIG. 4 illustrates an example graph of the detected light signal for red and IR signals for oxygenated blood and deoxygenated blood;
FIG. 5 illustrates an example graph of oxygen saturated levels based on a calculated ratio in accordance with example embodiments of the present disclosure;
FIG. 6 illustrates an example graph of the detected light signal for green, red, and IR light as detected by a photodetector in accordance with example embodiments of the present disclosure;
FIG. 7 illustrates a flowchart depicting an example process of estimated oxygen levels in the blood of a user in accordance with example embodiments of the present disclosure; and
FIG. 8 illustrates example inputs to a confidence model according to example embodiments of the present disclosure.
FIG. 9 illustrates an example computing environment including a wearable computing device in accordance with example embodiments of the present disclosure.
FIG. 10 depicts an example flow diagram for a method for estimating oxygen levels in the blood of a user in accordance with example embodiments of the present disclosure.

DETAILED DESCRIPTION

Reference now will be made in detail to embodiments, one or more examples of which are illustrated in the drawings. Each example is provided by way of explanation of the embodiments, not limitation of the present disclosure. In fact, it will be apparent to those skilled in the art that various modifications and variations can be made to the embodiments without departing from the scope of the claims. For instance, features illustrated or described as part of one embodiment can be used with another embodiment to yield a still further embodiment.

Generally, the present disclosure is directed towards a system for accurately estimating oxygen saturation levels in the blood of a user by non-invasively measuring at the wrist of a user by projecting light at the wrist and measuring the reflected light. To do so, a wearable computing device (e.g., such as a fitness band) can be positioned around the wrist of a user. The wearable computing device can include one or more components for projecting light with a certain wavelength towards the skin of a user. A photodetector (e.g., a photodiode) can measure the intensity of the light that is reflected (or emitting) from the user and generate a corresponding electric signal. However, due to one or more factors (including a relatively low density of capillaries present in the wrist of users, instances of low perfusion due to low temperature, and so on), the corresponding electric signal can be below a target for accuracy.

To improve the accuracy of generated electric signals with respect to the oxygenation of the blood, the wearable computing device can gather data for red, green, and infrared light. The wearable computing device can generate an initial estimate of the saturation of the blood of the user based on a comparison of the intensity or amount of light emitted in each wavelength range. Specifically, the wearable computing device can determine a ratio between measured intensity of light in the red light wavelength spectrum and of the measured intensity of light in the infrared wavelength spectrum. The wearable computing device can use the measured green light wavelength intensity to filter the signals produced for the intensity of the red light and the intensity of the infrared light. Specifically, the wearable computing device can calculate a dot product of the electric signal representing the intensity of green light with both the electric signal representing the intensity of red light and the electric signal representing the intensity of infrared light. This can have the effect of using the green light signal to filter the red light signal and the infrared light signal to remove the noise caused by several different factors. It can also have the effect of enabling the system to accurately measure the saturation levels at a consistent time within the user's heartbeat. The filtered red light signal and the filtered infrared light signal can be used to generate a more accurate estimated saturation.

In some examples, the wearable computing device can generate one or more features from the filtered signals. Features can include information such as the intensity of the measured green light, the intensity of the measured red light, the intensity of the measured infrared light, the ratios between the signals generated by the photodiode, the dot product of two or more electric signals based on the intensity of one or more wavelengths of lights, the average values over a one second period, and so on. These features can be input into a machine-learned model. The machine-learned model can be trained to provide a confidence value for the estimated oxygen saturation level for the blood of a user. In some examples, the wearable computing device generates an estimated classification for the blood as saturated or unsaturated. The output of the machine learning model can represent a confidence level that this estimated classification is correct. In some examples, the estimated saturation value is in a range of values from completely unsaturated to fully saturated, with a plurality of values within that range. If the estimated saturation level is represented as a value within a range, the confidence value can be a representation of whether the estimated saturation level is within an appropriate range.

For example, a user can have a wearable computing device on their wrist (e.g., a fitness band), the wearable computing device can include three LEDs. A first LED can produce light in the red light spectrum, a second LED can produce light in the green light spectrum, and a third LED can produce light in the infrared light spectrum. A photodiode can detect light emitted from the user's skin in each associated wavelength. The detected light in one or more of the light wavelengths can be compared to generate an initial saturation estimation. The initial saturation estimation can be that the user's blood is saturated with oxygen. A plurality of features can be extracted from the signals generated by the photodiode. Each feature can be provided to the machine-learned model as input. The machine-learned model can generate an output value that represents a high confidence that the initial estimation is correct. Based on the high confidence value, the wearable computing device can provide the estimated saturation value to the user (or use that value in other calculations).

More specifically, a wearable computing device can include any computing device that is integrated into an object that is meant to be worn by a user. For example, wearable computing devices can include, but are not limited to smartwatches, fitness bands, computing devices integrated into jewelry such as smart rings or smart necklaces, computing devices integrated into items of clothing such as jackets, shoes, and pants, and wearable glasses with computing elements included therein. In some examples, a wearable computing device can include one or more sensors intended to gather information with the permission of the user that is wearing the wearable computing device. Such information can include, but is not limited to, heart rate, body composition, sweat level, hydration level, blood oxygen saturation levels, and so on

The wearable computing device can include one or more light sources and one or more light detecting components. In some examples, the light sources can be light emitting diodes (LEDs) and they can be configured such that they produce light in one or more specified wavelength ranges. For example, a particular wearable computing device can include one or more red LEDs, one or more green LEDs, and one or more infrared LEDs. Each LED can generate light in its associated wavelengths. In some examples, the light generated by each LED can be generated within a narrow band of wavelengths. For example, the one or more green LEDs can generate light that has a wavelength between 500 nm and 565 nm. The one or more red LEDs can generate light that has a wavelength between 625 nm and 750 nm. The one or more infrared LEDs can generate light that has a wavelength greater than 750 nm.

The wearable computing device can also include a sensor. The sensor can be a light sensor that can detect light emitted from the skin of a user. In some examples, the light sensor can be a broadband photodiode. The photodiode can detect light in a variety of wavelengths. In some examples, the photodiode can determine the specific wavelengths that are present and the intensity of light at each of the present wavelengths. The photodiode can transform the detected light into electric signals. In some examples, the photodiode can collect all three wavelengths simultaneously and use signal processing techniques to extract the different signals associated with different wavelengths. In other examples, each LED can be turned on for a brief period in succession and the resulting signal from that period can be associated with a specific LED.

The wearable computing device can use the generated electric signals to estimate oxygen saturation levels of the user's blood. The estimated oxygen saturation levels can be used to determine whether the user's blood is oxygenated or deoxygenated. For example, oxygenated blood can absorb less red light and as a result, more red light is reflected. Thus, the photodiode can detect emitted (or reflected) red light at a higher intensity. The resulting electric signal generated in response to the red light is higher. Conversely, when the blood is less oxygenated, the blood absorbs less infrared light and thus, the electric signal produced in response to infrared light is higher.

Using these signals, the wearable computing device can generate a saturation ratio. The saturation ratio can represent the ratio of the electric signal associated with red light to the electric signal associated with infrared light. The higher the value of the saturation ratio, the more likely the user's blood is saturated with oxygen. In some examples, a predetermined threshold can be used to determine whether the user's blood is saturated or not. If the saturation ratio exceeds the predetermined threshold, the wearable computing device can determine that the blood is saturated. If the saturation ratio does not exceed the predetermined threshold, the wearable computing device can determine that the user's blood is not saturated with oxygen.

In some examples, one or more factors can reduce the accuracy of the saturation ratio. Specifically, low perfusion (e.g., low amounts of blood in a particular area of the body) can reduce the accuracy of the measured red and infrared light (due to less total blood) and can, in turn, result in the saturation ratio being less accurate. Low perfusion can be the result of the user's heartbeat, respiration (especially as a result of rapid or strong respiration), venous pulsations (e.g., movement of the muscles near the target area), changes in temperature (e.g., colder temperatures may reduce the blood at the extremities), and the density of capillaries at the target area.

In some examples, additional factors can include Meyer waves, which are oscillations of arterial pressure occurring spontaneously in conscious users at a frequency lower than respiration. The intensity of light measured can also be affected based on the density of capillaries present in the portion of the user's body at which the measurement is taken. Venous blood can be deoxygenated relative to arterial blood. As such, areas of the body that have a high density of veins can make the oxygenation of blood more difficult to determine.

The issues with the reliability of the saturation ratio are resolved by filtering the electric signals associated with the measured red light and the measured infrared light. To do so, the wearable computing device generates a dot product value for the red light and the green light time series data as the first dot product value. The wearable computing device generates a dot product from the infrared light and the green light time series data as a second dot product value.

Because the detected green light has higher perfusion than red or IR light and a high signal-to-noise ratio at the heartbeat, generating a dot product with the green light series data can have the effect of filtering the red/IR data to frequencies corresponding to the heartbeat of the user. In this way, the first and second dot product values can be used to determine the amplitude of the red/IR waveform at each heartbeat. This is useful because the heartbeat can correspond to points in time at which perfusion is highest and thus provides the most reliable measure of oxygen in the user's blood. This also allows the system to consistently measure arterial blood oxygenation (e.g., new blood enters the artery at each heartbeat as it is pumped through the system).

In some examples, the detected green light can be used to filter the red and infrared light data in other ways. Specifically, the filtering system can extract the user's heart rate based on an analysis of the green light data. The known heart rate frequency can be used to tune a frequency selective filter. That frequency selective filter can be applied to the red and infrared light data to result in increased accuracy (by removing data with higher noise).

Alternatively, the filtering system can determine the heart rate of the user based on the green light signal data. That measured heart rate can be used to create a bandpass filter based on that heart rate. The bandpass filter can then filter the red and infrared light signal data. In another alternatively, the filtering system can use fast Fourier transform (FFT) to determine a frequency peak for the green light signal data (or peaks). The filter system can then use these peaks to compute a dot product with each of the read light signal data and the infrared light signal data using the data from the peak to create a relatively noiseless filtered signal. In some examples, the peak data can be used as timing data to select information from the red light signal data and the infrared light signal data.

In some examples, once the wearable computing device has determined a saturation ratio based on filtered time-series data (or otherwise processed the data to improve the reliability of the saturation ratio), the wearable computing device can generate feature data from the time-series signal data (either directly or by processing the electric signal data). Features can include, but are not limited to, correlations between red light or IR and green light and the ratio of red perfusion to green perfusion. Specifically features that can be used as input to the classifier can include the mean value for the saturation coefficient, the red signal and IR signal slope, the red-green correlation coefficient, the red perfusion/green perfusion value, the red IR correlation coefficients, the saturation ratio, the square and cube of the saturation ratio, the correlation between the red signal and the IR signal, the correlation between the red and the green signals, the correlation between the green and IR signals, the ratio of red perfusion to green perfusion, an IR perfusion value, a red perfusion value, the AC ratio mean, the DC ratio mean, the standard deviation of the acceleration, the green response power, the red AC amplitude variation divided by the IC AC amplitude variation, the red DC slope and absolute values, the IR DC slope and absolute values, the red DC standard deviation, and the IR DC standard deviation.

The features can be used as input to a machine-learned model. The machine-learned model can be trained to take input associated with the time series data generated based on the detected light and output a confidence value. The confidence value can represent the degree to which the model is confident that the estimated saturation is accurate. For example, if the wearable computing device has estimated that the blood is saturated, and the confidence value output by the machine-learned model is high, the wearable computing device can determine that the initial estimation is likely accurate. Similarly, if the initial estimation is that the blood is unsaturated, and the confidence value is high, the wearable computing device can determine that the initial estimate of unsaturated blood is likely accurate.

In some examples, the machine-learning model can output a low confidence value. A low confidence value can represent that the initial estimation with respect to saturation cannot be verified with high confidence. In some examples, if the confidence value is below a lower threshold, the saturation estimation can be discarded altogether, and a new measurement can be taken.

In some examples, the oxygen saturation level of the user's blood can be presented to the user in the user interface associated with the wearable computing device. For example, if the wearable computing device is a smartwatch, the oxygen saturation value can be displayed in a portion of the user interface of the display of the smartwatch. In some examples, measurement of blood saturation is done during periods of sleep such that the data is averaged over a long period. In other examples, measurements of blood saturation can be performed upon the request of the user.

Embodiments of the disclosed technology provide a number of technical effects and benefits, particularly in the areas of wearable computing devices. In particular, embodiments of the disclosed technology provide improved techniques for estimating oxygen saturation using green optical light as a filter. For example, utilizing embodiments of the disclosed technology, a wearable computing device can more accurately determine the oxygen saturation of a user's blood. Improving the accuracy can result in a better and more useful user experience. Furthermore, this effect is accomplished with relatively little cost. As such, the disclosed embodiments enable additional functionality without significantly increasing the total cost of a wearable device.

With reference now to the figures, example aspects of the present disclosure will be discussed in greater detail.

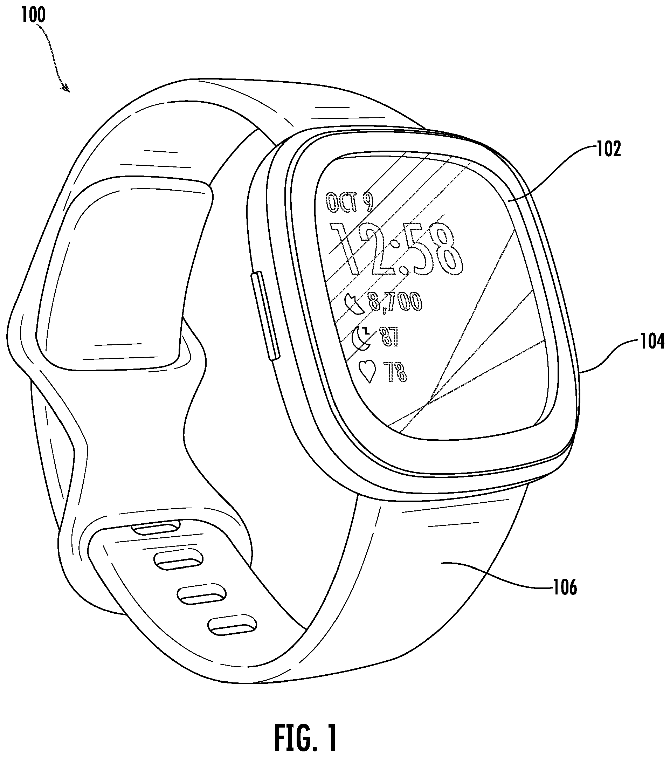

FIG. 1 depicts the front view of an example wearable device 100 according to example embodiments of the present disclosure. In a particular example, the wearable device 100 may be a wristband, a bracelet, a wristwatch, an armband, a ring placed around a digit of the user, or other wearable products that may be equipped with sensors as described in the present disclosure. The wearable device 100 can be configured with a display 102, a device housing 104, and a band 106. The display 102 can be configured to present to a user data relating to the user's oxygen saturation level, skin temperature, heart rate, electroencephalograms, electrocardiograms, electromyography, electrooculograms, and other physiological data of the user (e.g., blood oxygen level). The display 102 can also be configured to convey data from additional ambient sensors contained within the wearable device 100. Example information conveyed on the display 102 from these additional ambient sensors can include a positioning, altitude, and weather of a location associated with the user. The display 102 can also convey data regarding the motion of the user (e.g., whether the user is stationary, walking, and/or running).

In an embodiment, the display 102 can be configured to receive data input by the user. In some examples, the wearable computing device can be configured to measure the oxygen saturation in the blood of the user who is wearing the wearable computing device 100. To do so, the wearable computing device 100 can include one or more LEDs to project light towards the skin of the user and a photodiode to measure any light that is emitted back from the skin. In some examples, the LEDs can be configured to project light toward the skin of a user over a prolonged period (e.g., while the user is sleeping).

Using the interface displayed on the display 102, the wearable computing device 100 can display the average oxygen saturation for the user during a period of sleep. In some examples, a user can, by input on the display, request that the wearable computing device 100 generate additional data for display to the user (e.g., oxygen saturation data). In response, the display can present instructions to the user (e.g., instructions to position the wearable computing device 100 properly) to obtain the data requested. Furthermore, if, while the additional data is being gathered, the wearable computing device 100 determines that additional data is necessary, the display 102 can display instructions to the user (e.g., please remain still for 10 seconds).

In an embodiment, the device housing 104 can be configured to contain one or more sensors described in the present disclosure. Example sensors contained by the device housing 104 can include light sensors (e.g., photodiodes), skin temperature sensors, internal device temperature sensors, location sensors (e.g., GPS), motion sensors, altitude sensors, heart rate sensors, audio sensors, pressure sensors, and other physiological sensors. In an embodiment, the device housing 104 can also be configured to include one or more processors. The band 106 can be configured to secure the wearable device 100 around an arm of a user by, for example, connecting the ends of the band 106 with a buckle, clasp, or other similar securing device, thereby allowing the wearable device 100 to be worn by the user.

The wearable computing device 100 can include one or more light emitting diodes (LEDs). Each LED can be a semiconductor diode that produces light at a specific wavelength or frequency when voltage is applied. The specific wavelengths can be selected to be useful in determining whether or not the blood in the user's body is saturated with oxygen. In this example, the LEDs can include an LED configured to produce red light, an LED configured to produce infrared light, and an LED configured to produce green light. The LEDs can be positioned within the wearable computing device 100 such that they project their light downward into the skin of a user. The LEDs can also be configured to be positioned such that the light projected by the LEDs can be reflected (or absorbed and emitted) back to a photodiode sensor in the wearable computing device 100.

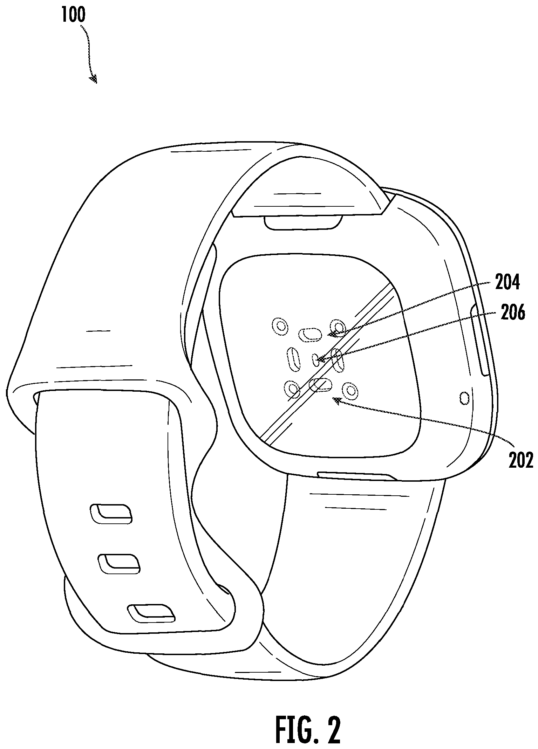

FIG. 2 illustrates an example of a rear side of a wearable computing device 100 in accordance with example embodiments of the present disclosure. The wearable computing device 100 can include one or more red, green, and IR LEDs 202 and a broadband photodiode 204. In some examples, the LEDs can include additional green LEDs 206. These LEDs can be turned on sequentially (e.g., one after another such that only one is active at a given time). If so, each LED can be activated for a very limited period. In some examples, the broadband photodiode 204 can generate time series data representing the intensity of the light measured at the associated wavelength for a plurality of points in time. For example, the time series data can include, for a series of points, corresponding values for the intensity of each of the wavelengths of light projected by the LEDs. For example, at time 1, the photodiode can generate an intensity value for the red light, an intensity value for the infrared light, and an intensity value for the green light.

In some examples, the followed by all can measure the intensity of light by automatically converting the light into an electric signal. The electric signal can be measured and converted into a value that can be stored in a time series list of values. In some examples, the electric signals can include an alternating current portion of the signal (AC signal) and a direct current portion of the signal (DC signal).

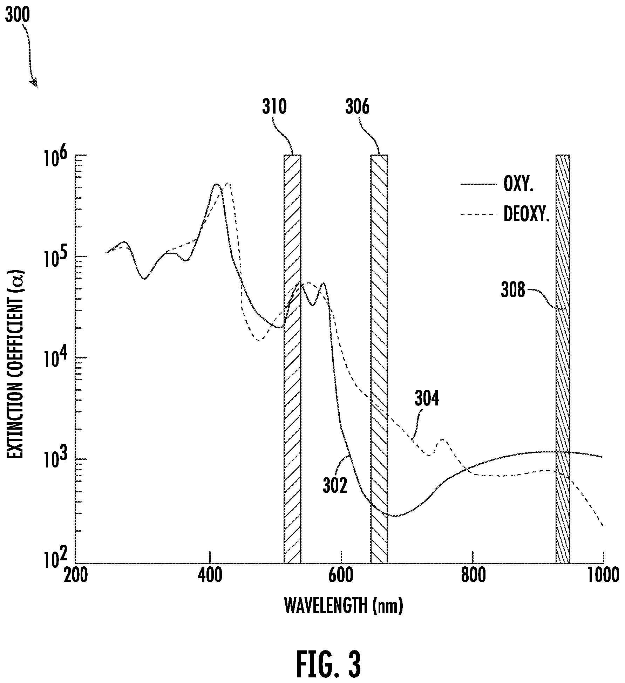

FIG.3 illustrates an example graph 300 representing an expected intensity of back-reflected light based on whether blood is oxygenated or deoxygenated. As can be seen, oxygenated blood and deoxygenated blood can have different extinction coefficients. An extinction coefficient can determine how strongly a material absorbs or reflects light at a particular wavelength.

In this example, both oxygenated blood 302 and deoxygenated blood 304 have a similar extinction coefficient in the green wavelengths 310. In contrast, within the red wavelengths 306, deoxygenated blood has a higher extinction coefficient than oxygenated blood. Within infrared wavelengths 308, deoxygenated blood has a lower extinction coefficient than oxygenated blood. Using these differences, the user computing system 100 can estimate whether the user's blood is oxygenated or deoxygenated.

For example, if the amount of red light reflected is greater than the amount of infrared light reflected, the wearable computing device can estimate that the blood is saturated with oxygen. Conversely, if the amount of red light reflected is less than the amount of infrared light reflected, the wearable computing device can estimate that the blood is deoxygenated.

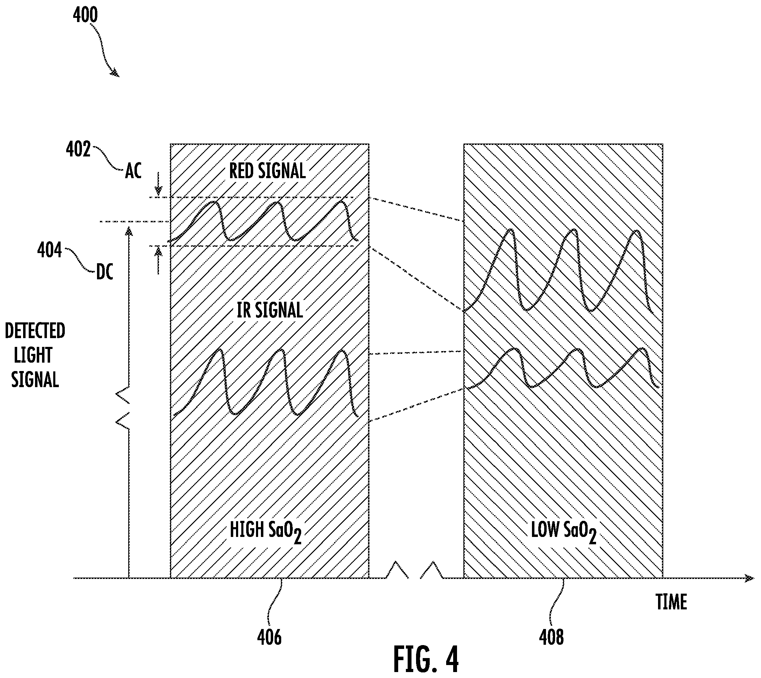

FIG. 4 illustrates an example graph 400 of the detected light signal for red and IR signals for oxygenated blood and deoxygenated blood. The electric signal for each wavelength of light can include an alternating current component 402 (AC) that represents changes in amplitude of the measured light (e.g., due to changes in the volume or flow of blood associated with the heartbeat or other factors). The direct current portion 404 (DC) of the electric signal can remain relatively constant if the oxygenation level of the blood does not change. A ratio of the AC signal to DC signal for the electric signal corresponding to red light can be divided by the ratio of AC signal to DC signal for the electric signal corresponding to infrared light to generate a saturation ratio (R). For example, R can be calculated as follows:

$$R = \frac{(AC/DC)_{red}}{(AC/DC)_{IR}}$$

The values for the AC signal and the DC signal for red and IR light can vary based on whether the user's blood is oxygenated 406 or deoxygenated 408. Thus, light with wavelengths in the infrared spectrum light is absorbed more (e.g., less light reflected and thus smaller DC signal) and is more sensitive to changes in blood volume when the blood being measured is highly oxygenated (larger AC signal amplitude). When the blood is less oxygenated, the infrared light is absorbed less (e.g., it has a higher amount of reflected light measured and thus a higher DC signal) and is less sensitive to changes in blood volume (e.g., the change in intensity of the measured light is reduced and thus less AC signal amplitude).

Conversely, light with wavelengths in the red spectrum is absorbed less (e.g., more light reflected giving a larger DC signal) and less sensitive to changes in blood volume when the blood being measured is highly oxygenated (e.g., less AC signal amplitude). When the blood is less oxygenated, the red light is absorbed more (e.g., less light is detected reflecting to the photodetector and thus a smaller DC signal) and is more sensitive to changes in blood volume (e.g., the change in intensity of the measured light is increased and thus the AC signal amplitude increases).

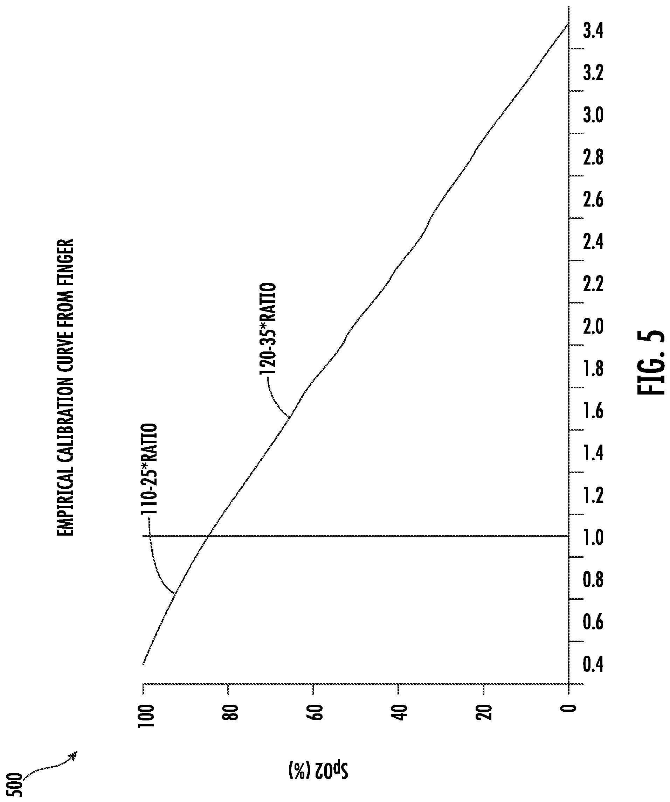

FIG. 5 illustrates an example graph 500 of oxygen saturation levels based on a calculated ratio in accordance with example embodiments of the present disclosure.

In some examples, one or more factors can make an estimation of blood oxygenation levels less accurate. For example, respiration can affect the estimated oxygen saturation value. This is especially true if breathing is too rapid (or frequent) or strong. Similarly, the movement of the user can cause venous pulsations (e.g., mechanical pulsations of the veins) which alters the amount of blood in the area or the movement of the blood, making accurate estimation more difficult.

Low perfusion (e.g., reduced blood due to lowered temperate or other factors) can reduce the accuracy of blood oxygen estimation. In some examples, additional factors can include Meyer waves, which are oscillations of arterial pressure occurring spontaneously in conscious users at a frequency lower than respiration. The intensity of light measured can also be affected based on the density of capillaries present in the portion of the user's body at which the measurement is being taken. Venous blood can be deoxygenated relative to arterial blood. As such, areas of the body that have a high density of veins can make the oxygenation of blood more difficult to determine.

For example, as noted above, fingertips can have a greater density of capillaries than the wrist of a user. Using only the red and infrared light spectrums to estimate the amount of oxygen present in the user's blood can result in inaccurate measurements because of one or more of these different factors.

To mitigate these factors, the wearable computing device can generate a measurement of oxygen in the user's blood that is independent of these factors. One method for doing so is to use a third wavelength of light. In this case, a third wavelength of light can be in the green wavelength spectrum.

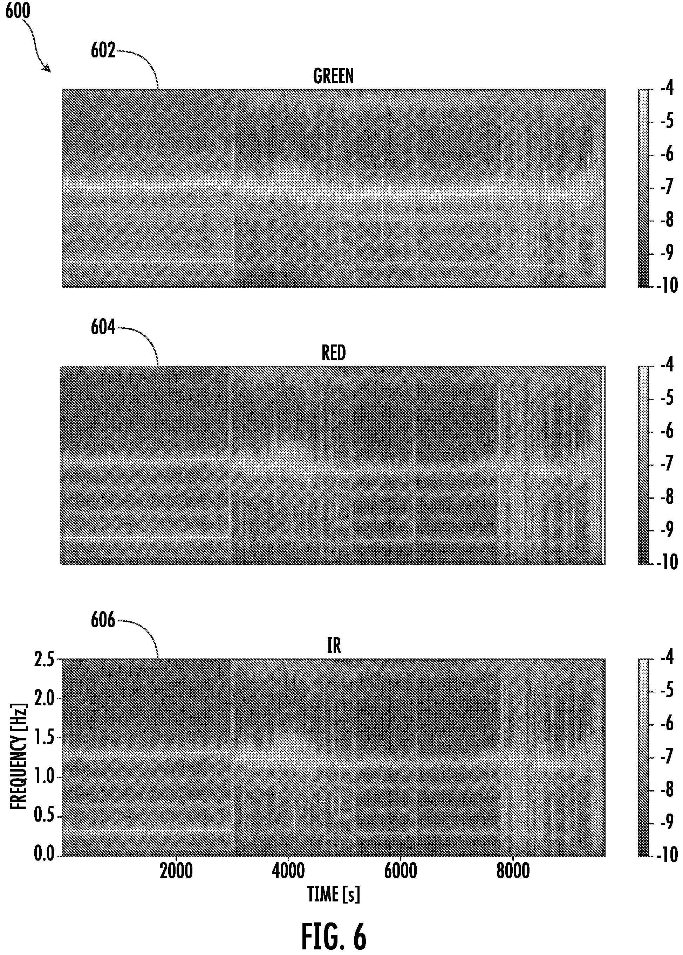

FIG. 6 illustrates an example graph 600 of the detected light signal for green, red, and IR light as detected by a photodetector in accordance with example embodiments of the present disclosure. Green light 602 itself does not necessarily demonstrate a notable difference in intensity between oxygenated and deoxygenated blood that is accurate for determining oxygen levels. However, the green light 602 can have a high signal-to-noise ratio at a frequency corresponding to a user's pulse rate.

Thus, the wearable computing device can include one or more red light sources (e.g., one or more LEDs emitting light in the red light spectrum), an infrared light source (e.g., one or more LEDs emitting light in the infrared light spectrum), and one or more green light source (e.g., one or more LEDs emitting light in the green light spectrum), and one or more photodetectors (e.g., one or more broadband photodiodes). The wearable computing device can be operated such that each of the three light sources is turned on sequentially. However, in some cases, the light sources may be at least partially activated at the same time. The photodetector can measure the light reflected from the skin of the user associated with the wavelength of light that was being emitted at the time that the light was detected.

Thus, for each of the light sources, the photodetector can generate time series data representing the intensity of the light measured at the associated wavelength for a plurality of points in time. The wearable computing device can generate a dot product value for the red light 604 and the green light 602 time series data as the first dot product value. The wearable computing device can generate a dot product from the infrared light 606 and the green light 602 time series data as a second dot product value.

Because the detected green light 602 has higher perfusion than red or IR light (604 and 606 respectively) and a high signal-to-noise ratio at the heartbeat, generating a dot product with the green light series data can have the effect of filtering the red/IR data to frequencies corresponding to the heartbeat of the user. In this way, the first and second dot product values can be used to determine the amplitude of the red/IR waveform at each heartbeat. This is useful because the heartbeat can correspond to points in time at which perfusion is highest and thus provides the most reliable measure of oxygen in the user's blood. This also allows the system to consistently measure the arterial blood oxygenation (e.g., new blood enters the artery at each heartbeat as it is pumped through the system).

In some examples, rather than calculating the dot product between the red/IR light series and the green light series data, another method can be used. For example, a bandpass filter can be tuned to the heart rate frequency found in the green light series data.

In some examples, the user computing system can include one or more machine-learned models. The one or more machine-learned models can enable the system to determine a confidence level associated with the estimated oxygen saturation level in the blood of a user.

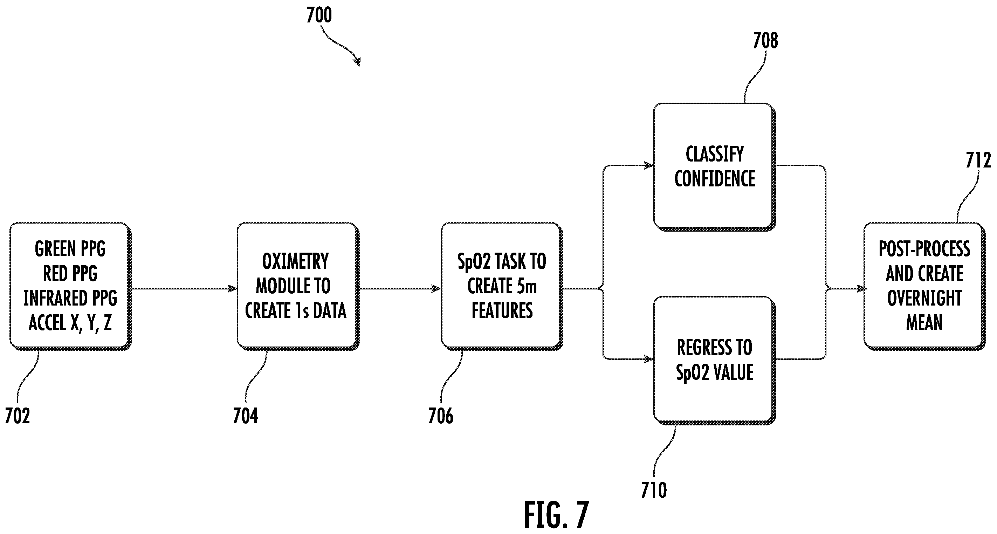

FIG. 7 illustrates a flowchart depicting an example process of estimating oxygen levels in the blood of a user in accordance with example embodiments of the present disclosure. One or more portion(s) of the method can be implemented by one or more computing devices such as, for example, the computing devices described herein. Moreover, one or more portion(s) of the method can be implemented as an algorithm on the hardware components of the device(s) described herein. FIG. 7 depicts elements performed in a particular order for purposes of illustration and discussion. Those of ordinary skill in the art, using the disclosures provided herein, will understand that the elements of any of the methods discussed herein can be adapted, rearranged, expanded, omitted, combined, and/or modified in various ways without deviating from the scope of the present disclosure. The method can be implemented by one or more computing devices, such as one or more of the computing devices depicted in FIGS. 1 and 2.

In some examples, the wearable computing device (e.g., wearable computing device 100 in FIG. 1), can, at 702, use photoplethysmography data for green light, red light, and infrared light as input. In some examples, the green light, red light, and infrared light are projected onto the skin of the user wearing the wearable computing device 100. The wearable computing device 100 can be a wearable smartwatch. The wearable computing device 100 can include one or more LEDs. The LEDs can be controlled such that they produce light in the wavelength based on their configuration in response to a voltage being applied. In some examples, the wearable computing device 100 can include other light sources.

The wearable computing device 100 can include a photodiode that detects light reflected from the user's skin. In some examples, the photodiode (or another light sensor) can generate an electric signal that represents the detected light, in one or more wavelengths.

In addition, movement data from an accelerometer can be used as input. An oximetry module can generate, at 704, oxygen saturation data for a one second period. The wearable computing device 100 can use data for a five-minute period (based on a series of one second data slices) to extract feature data, at 706, for the five-minute period. For example, the wearable computing device 100 can collect a series of one second slices over a five-minute period. These slices can then be averaged or summed to obtain a number representing the entire period. Other analysis and signal processing techniques can be performed to extract features from the time series data representing the intensity of wavelengths of light over time.

The wearable computing device 100 can use a regression model to determine an estimated oxygen saturation value at 710. The regression model can perform a regression analysis to determine an estimated oxygen saturation level for the blood of the user.

The wearable computing device 100 can use a machine-learned model, at 708, to determine a confidence value representing the degree to which the input data can be used to generate an accurate estimation of oxygen saturation for the user. For example, the machine-learned model can be trained such that the machine-learned model can take a plurality of features as input. The machine-learned model can output a confidence value. The confidence value can represent the degree to which the estimated oxygen saturation level is accurate.

The wearable computing device 100 can process the confidence value and the estimated oxygen saturation from a plurality of five-minute periods to generate, at 712, an overnight mean value for oxygen saturation.

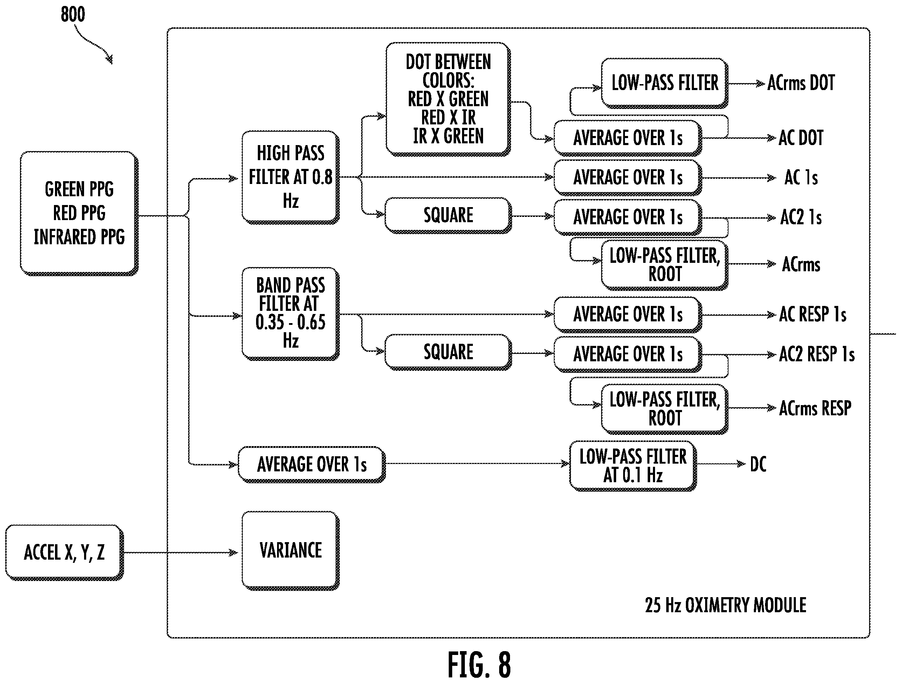

FIG. 8 illustrates example inputs 800 to a confidence model according to example embodiments of the present disclosure. For example, the confidence classifier can use a variety of input signals including correlations between red light or IR and green light and the ratio of red perfusion to green perfusion.

Features that can be used as input to the classifier can include, but are not limited to, the mean value for the saturation coefficient, the red signal and IR signal slope, the red/green correlation coefficient, the red perfusion/green perfusion value, the red IR correlation coefficients, the saturation ratio, the square and cube of the saturation ratio, the correlation between the red signal and the IR signal, the correlation between the red and the green signals, the correlation between the green and IR signals, the ratio of red perfusion to green perfusion, an IR perfusion value, a red perfusion value, the AC ratio mean, the DC ratio mean, the standard deviation of the acceleration, the green response power, the red AC amplitude variation divided by the IC AC amplitude variation, the red DC slope and absolute values, the IR DC slope and absolute values, the red DC standard deviation, and the IR DC standard deviation. Other values can be used as input to the classifier to determine a confidence level associated with an oxygen saturation estimate.

Using a priority of measurements as inputs, the machine-learned confidence classifier can generate a confidence value. The confidence value can represent the degree to which the system is confident that the estimated oxygen saturation value is correct. In some examples, if the confidence value falls below a threshold, the oxygen saturation values can be discarded and not used further. If the confidence levels are above a certain predetermined threshold, the oxygen saturation values can be used (e.g., for display to a user or in other calculations).

In some examples, the wearable computing device can determine oxygen saturation using a machine-learned regression model instead of a lookup table based on the saturation ratio. The machine-learned regression model can use various inputs including, but not limited to the mean value for the saturation coefficient, the red signal and IR signal slope, the red/green correlation coefficient, the red perfusion/green perfusion value, the red IR correlation coefficients.

The user computing system can estimate the movement of the user as input to both the machine learning regression model and the machine learning confidence classifier. In some examples, an accelerometer can be used to determine the movement of a user. In other examples, the photoplethysmography (PPG) for the red light and the PPG for the infrared light can be used to determine the movement of the user. For example, movements that are difficult for an accelerometer to detect such as the flexing of an arm muscle can be detectable via PPG of the red light or infrared light and can be used to determine the confidence associated with saturation estimate.

In some examples, the saturation level of the users can be determined over a long period such as multiple minutes, hours, or days. In some examples, the user's oxygen saturation levels can be displayed to the user via a display incorporated into the wearable computing device.

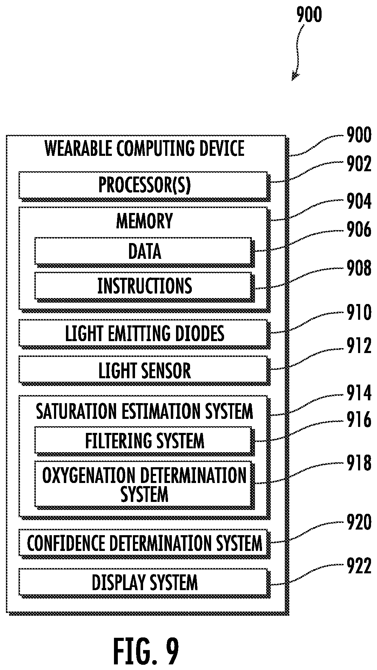

FIG. 9 illustrates an example computing environment including a wearable computing device 900 in accordance with example embodiments of the present disclosure. In this example, the wearable computing device 900 can include one or more processors 902, memory 904, one or more light emitting diodes 910, a light sensor 912, a saturation estimation system 914, a confidence determination system 920, and a display system 922.

In more detail, the one or more processors 902 can be any suitable processing device that can be embedded in the form factor of a wearable computing device. For example, such a processor can include one or more of: one or more processor cores, a microprocessor, an ASIC, a FPGA, a controller, a microcontroller, etc. The one or more processors can be one processor or a plurality of processors that are operatively connected. The memory 904 can include one or more non-transitory computer-readable storage mediums, such as RAM, ROM, EEPROM, EPROM, flash memory devices, etc., and combinations thereof.

In particular, in some devices, memory 904 can store instructions for implementing the saturation estimation system 914, the confidence determination system 920, and the display system 922. The wearable computing device 900 can implement the saturation estimation system 914 to execute aspects of the present disclosure.

It will be appreciated that the term "system" can refer to specialized hardware, computer logic that executes on a more general processor, or some combination thereof. Thus, a system can be implemented in hardware, application specific circuits, firmware and/or software controlling a general-purpose processor. In one embodiment, the system can be implemented as program code files stored on the storage device, loaded into memory, and executed by a processor or can be provided from computer program products, for example computer executable instructions, that are stored in a tangible computer-readable storage medium such as RAM, hard disk or optical or magnetic media.

Memory 904 can also include data 906 and instructions 908 that can be retrieved, manipulated, created, or stored by the one or more processor(s) 902. In some example embodiments, such data can be accessed and used as input to the saturation estimation system 914, the confidence determination system 920, and the display system 922. In some examples, the memory 904 can include data used to perform one or more processes and instructions that describe how those processes can be performed.

The wearable computing device 900 can include one or more light sources (e.g., a light emitting diode 910) and one or more light detecting components (e.g., a light sensor 912). In some examples, the light sources can be light emitting diodes (LEDs) and they can be tuned such that they produce light in one or more specified wavelength ranges. For example, a particular wearable computing device 900 can include a red LED, a green LED, and an infrared LED. Each LED can generate light in associated wavelengths which it is configured to produce. In some examples, the light generated by each LED can be fairly narrowly generated within a narrow band of wavelengths. For example, the green LED can generate light that has a wavelength between 500 nm and 565 nm. The red LED can generate light that has a wavelength between 625 nm and 750 nm. The infrared LED can generate light that has a wavelength greater than 750 nm.

The wearable computing device 100 can also include a light sensor 912. The light sensor can be a sensor that can detect light emitted from the skin of a user. In some examples, the light sensor 912 can be a photodiode. The photodiode can detect light in a variety of wavelengths. In some examples, the photodiode can determine the specific wavelengths that are present and the intensity of light at each of the present wavelengths. The photodiode can transform the detected light into electric signals. In some examples, the photodiode can collect light in all three wavelengths simultaneously and the wearable computing device 100 can use signal processing techniques to extract the different signals from different wavelengths. In other examples, each LED can be turned on for a brief period in succession and the resulting signal from that period of time can be associated with a specific LED.

The wearable computing device 900 can use the electric signals generated to estimate the oxygen saturation levels of the user's blood. For example, oxygenated blood can absorb less red light. Thus, the photodiode detects emitted (or reflected) red light at a higher intensity. Thus, the electric signal generated in response to the red light is greater. Conversely, when the blood is less oxygenated, the blood absorbs less infrared light and thus, the electric signal produced in response to infrared light is greater.

The electric signals produced by the light sensor 912 are accessed by the saturation estimation system 914. The saturation estimation system 914 can determine a saturation ratio for the signals. In some examples, the saturation ratio is the ratio of the red light electric signals to the infrared electric signals. A high saturation ratio can be associated with oxygenated blood while a low saturation ratio can be associated with deoxygenated blood.

The filtering system 916 can improve the reliability of the saturation ratio by filtering the electric signals associated with the measured red light and the measured infrared light. To do so, the wearable computing device can generate a dot product value for the red light and the green light time series data as the first dot product value. The filter system 916 can generate a dot product from the infrared light and the green light time series data as a second dot product value.

Generating a dot product with the green light series data can have the effect of filtering the red/IR data to frequencies corresponding to the heartbeat of the user. In this way, the first and second dot product values can be used to determine the amplitude of the red/IR waveform at each heartbeat. This is useful because the heartbeat can correspond to points in time at which perfusion is highest and thus provides the most reliable measure of oxygen in the user's blood. This also allows the system to consistently measure the arterial blood oxygenation (e.g., new blood enters the artery at each heartbeat as it is pumped through the system).

In some examples, the oxygenation determination system 918 can determine an estimated oxygenation level for the user's blood. In some examples, an estimated oxygenation level can be one of either oxygenated or deoxygenated. For example, the wearable computing device can have a predetermined threshold value above which blood is considered oxygenated and below which blood is considered deoxygenated. In some examples, the oxygenation determination system 918 can provide a specific estimated oxygenation level which can be compared against the threshold. In other examples, the specific oxygen level can be stored and displayed to the user.

In some examples, once the saturation estimation system 914 has determined a saturation ratio and filtered the electric signal data (or otherwise processed the data to improve the reliability of the saturation ratio), the confidence determination system 920 can generate feature data from the electric signal data (either directly or by processing the electric signal data).

The features can be used as input to a machine-learned model by the confidence determination system 920. The machine-learned model can be trained to take input associated with the electric signals generated based on the detected light and output a confidence value. The confidence value can represent the degree to which the model is confident that the estimated saturation is accurate.

Once the counter determination system 920 has determined confidence for the estimated oxygenation level, the wearable computing device can access the display system 922. The display system 922 can control the display of the wearable computing device 900 and can display information of interest to the user on the display. Some examples come on the user can request specific information be displayed and the display system 922 can provide that information.

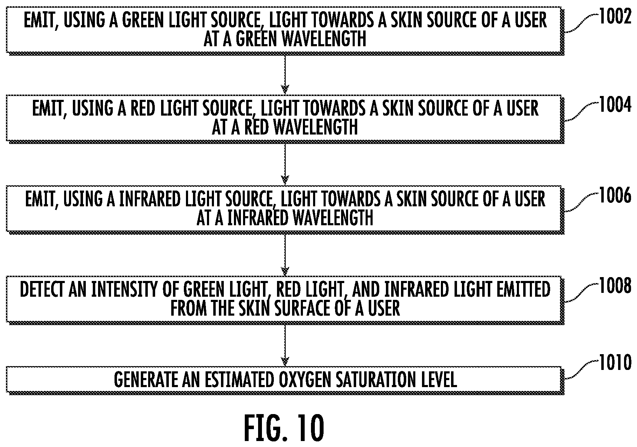

FIG. 10 depicts an example flow diagram for a method for estimating oxygen levels in the blood of a user in accordance with example embodiments of the present disclosure. One or more portion(s) of the method can be implemented by one or more computing devices such as, for example, the computing devices described herein. Moreover, one or more portion(s) of the method can be implemented as an algorithm on the hardware components of the device(s) described herein. FIG. 10 depicts elements performed in a particular order for purposes of illustration and discussion. Those of ordinary skill in the art, using the disclosures provided herein, will understand that the elements of any of the methods discussed herein can be adapted, rearranged, expanded, omitted, combined, and/or modified in various ways without deviating from the scope of the claims. The method can be implemented by one or more computing devices, such as one or more of the computing devices depicted in FIGS. 1-2.

A wearable computing device (e.g., wearable computing device 100 in FIG. 1) can include one or more processors, memory, and other components that, together, enable the wearable computing device 100 to estimate the oxygen saturation levels in the blood of a user. The wearable computing device 100 can, at 1002 emit, using one or more green light sources, light towards a skin of a user at a green wavelength. For example, the one or more green LEDs can generate light that has a wavelength between 500 nm and 565 nm. The wearable computing device 100 can, at 1004, emit, using one or more red light sources, light towards the skin of a user at a red wavelength. The one or more red LEDs can generate light that has a wavelength between 625 nm and 750 nm. The wearable computing device 100 can emit, at 1006, emit, with one or more using an infrared light source, light towards a skin of a user at an infrared wavelength.

The wearable computing device 100 can, at 1008, detect, by the wearable computing device 100, an intensity of green light, red light, and infrared light emitted from the skin surface of a user. In some examples, a photodiode is used to detect an intensity of green light, red light, and infrared light emitted from the skin surface of a user. The photodiode can generate, for each of green light, red light, and infrared light, a respective electric signal, the respective electric signal representing the intensity of the associated light over time.

The wearable computing device 100 can generate an estimated oxygen saturation level. To do so, the wearable computing device 100 can determine a saturation ratio between the electric signal for the red light and the electric signal for the infrared light.

The wearable computing device 100 can filter the electric signal for the red light and the electric signal for the infrared light using the electric signal associated with the green light. To do so, the wearable computing device 100 can generate a dot product of the electric signal associated with the green light and the electric signal associated with the red light. The wearable computing device 100 can generate a dot product of the electric signal associated with the green light and the electric signal associated with the infrared light.

In some examples, the wearable computing device 100 can generate feature data from the detected intensity of green light, red light, and infrared light. The wearable computing device 100 can determine, using a machine-learned model with the feature data as input, a confidence level associated with the estimated oxygen saturation level of the blood of the user.

The wearable computing device 100 can determine whether the confidence level exceeds a predetermined threshold. In response to determining, by the computing system, that the confidence level exceeds a predetermined threshold, storing the estimated oxygen saturation level. The wearable computing device 100 can, in response to determining, by the computing system, that the confidence level does not exceed a predetermined threshold, discarding, by the computing system, the estimated oxygen saturation level. In some examples, the wearable computing device 100 can display the estimated oxygen saturation level.

The technology discussed herein refers to sensors and other computer-based systems, as well as actions taken and information sent to and from such systems. One of ordinary skill in the art will recognize that the inherent flexibility of computer-based systems allows for a great variety of possible configurations, combinations, and divisions of tasks and functionality between and among components. For instance, processes discussed herein may be implemented using a single system or multiple systems working in combination. Databases and applications may be implemented on a single system or distributed across multiple systems. Distributed components may operate sequentially or in parallel.

While the present subject matter has been described in detail with respect to specific example embodiments thereof, it will be appreciated that those skilled in the art, upon attaining an understanding of the foregoing may readily produce alterations to, variations of, and equivalents to such embodiments. Accordingly, the above is by way of example rather than by way of limitation, and does not preclude inclusion of such modifications, variations and/or additions to the present subject matter as would be readily apparent to one of ordinary skill in the art.

## Claims

1. A computer implementable method for measuring blood oxygen saturation in a user, the method comprising:

   emitting, using one or more green light sources (202, 206, 910), light towards a skin of a user at a green wavelength;
   emitting, using one or more red light sources (202, 910), light towards a skin of a user at a red wavelength;
   emitting, using one or more infrared light sources (202, 910), light towards a skin of a user at an infrared wavelength;
   detecting, by one or more light sensors (204, 912), an intensity of green light, red light, and infrared light emitted from the skin of a user, wherein the one or more light sensors generate, for each of green light, red light, and infrared light, a respective electric signal over time, forming a time series of intensity values for each wavelength, the respective electric signal for a particular wavelength of light representing the intensity of the wavelength of light; **characterized in that** the method further comprises:
   filtering the electric signal for the red light and the electric signal for the infrared light using the electric signal associated with the green light by:

      generating a dot product of a time series of the electric signal associated with the green light and a time series of the electric signal associated with the red light, and
      generating a dot product of the time series of the electric signal associated with the green light and a time series of the electric signal associated with the infrared light; and
      using the filtered electrical signals to determine an estimated oxygen saturation level.

**2.** The method of claim 1, further comprising: generating feature data from the detected intensity of green light, red light, and infrared light.

**3.** The method of claim 2, wherein the feature data comprises correlations between red light or infrared and green light and/or the ratio of red perfusion to green perfusion.

**4.** The method of claim 2 or claim 3, further comprising: determining, using a machine-learned model with the feature data as input, a confidence level associated with the estimated oxygen saturation level of the blood of the user.

**5.** The method of claim 4, further comprising: determining whether the confidence level exceeds a predetermined threshold.

**6.** The method of claim 5, further comprising: in response to determining that the confidence level exceeds a predetermined threshold, storing the estimated oxygen saturation level.

**7.** The method of claim 5, further comprising: in response to determining that the confidence level does not exceed a predetermined threshold, discarding the estimated oxygen saturation level.

**8.** The method of any preceding claim, further comprising: displaying the estimated oxygen saturation level.

**9.** The method of any preceding claim, wherein the electric signals include an alternating current portion of the signal and a direct current portion of the signal.

**10.** The method of any preceding claim, wherein the light sources are LEDs.

**11.** The method of claim 10, wherein the one or more green LEDs generate light that has a wavelength between 500 nm and 565 nm, the one or more red LEDs generate light that has a wavelength between 625 nm and 750 nm, and/or the one or more infrared LEDs generate light that has a wavelength greater than 750 nm.

**12.** The method of any preceding claim, wherein the method is performed by a wearable computing device (100).

**13.** The method of claim 12, wherein the wearable computing device is a smartwatch.

**14.** A wearable computing device (100), the wearable computing device comprising:

one or more green light sources, one or more red light sources, and one or more infrared light sources (202, 206, 910); one or more light sensors (204, 912); one or more processors (902); and a computer-readable memory (904), wherein the computer-readable memory stores instructions that, when executed by the one or more processors, cause the wearable computing device to perform the method of any of claims 1 to 11 and 13.

**15.** Computer program instructions, optionally stored on a computer-readable medium, that, when executed by one or more computing devices of a device comprising one or more green light sources, one or more red light sources, one or more infrared light sources, and one or more light sensors, cause the one or more computing devices to perform the method of any of claims 1 to 13.

**Patentansprüche**

**1.** Computerimplementierbares Verfahren zum Messen der Blutsauerstoffsättigung bei einem Benutzer, wobei das Verfahren umfasst:

Aussenden von Licht mit einer grünen Wellenlänge auf die Haut eines Benutzers unter Verwendung einer oder mehrerer grüner Lichtquellen (202, 206, 910); Aussenden von Licht mit einer roten Wellenlänge auf die Haut eines Benutzers unter Verwendung einer oder mehrerer roter Lichtquellen (202, 910); Aussenden von Licht mit einer infraroten Wellenlänge auf die Haut eines Benutzers unter Verwendung einer oder mehrerer Infrarotlichtquellen (202, 910); Erfassen, durch einen oder mehrere Lichtsensoren (204, 912), einer Intensität von grünem Licht, rotem Licht und infrarotem Licht, das von der Haut eines Benutzers emittiert wird, wobei der eine oder die mehreren Lichtsensoren für jedes des grünen Lichts, roten Lichts und infraroten Lichts ein jeweiliges elektrisches Signal über die Zeit erzeugen, das eine Zeitreihe von Intensitätswerten für jede Wellenlänge bildet, wobei das jeweilige elektrische Signal für eine bestimmte Wellenlänge die Intensität dieser Wellenlänge darstellt; **dadurch gekennzeichnet, dass** das Verfahren ferner umfasst: Filtern des elektrischen Signals für das rote Licht und des elektrischen Signals für das infrarote Licht unter Verwendung des elektrischen Signals, das dem grünen Licht zugeordnet ist, durch:

Erzeugen eines Skalarprodukts aus einer Zeitreihe des elektrischen Signals, das dem grünen Licht zugeordnet ist, und einer Zeitreihe des elektrischen Signals, das dem roten Licht zugeordnet ist, und
Erzeugen eines Skalarprodukts aus der Zeitreihe des elektrischen Signals, das dem grünen Licht zugeordnet ist, und einer Zeitreihe des elektrischen Signals, das dem infraroten Licht zugeordnet ist; und
Verwenden der gefilterten elektrischen Signale zur Bestimmung eines geschätzten Sauerstoffsättigungswerts.

2. Verfahren nach Anspruch 1, ferner umfassend:
Erzeugen von Merkmalsdaten aus der erfassten Intensität von grünem Licht, rotem Licht und infrarotem Licht.

3. Verfahren nach Anspruch 2, wobei die Merkmalsdaten Korrelationen zwischen rotem Licht oder infrarotem Licht und grünem Licht und/oder das Verhältnis von roter Perfusion zu grüner Perfusion umfassen.

4. Verfahren nach Anspruch 2 oder 3, ferner umfassend:
Bestimmen eines Vertrauensniveaus, das dem geschätzten Sauerstoffsättigungsniveau des Blutes des Benutzers zugeordnet ist, unter Verwendung eines maschinell erlernten Modells mit den Merkmalsdaten als Eingabe.

5. Verfahren nach Anspruch 4, ferner umfassend:
Bestimmen, ob das Vertrauensniveau einen vorgegebenen Schwellenwert überschreitet.

6. Verfahren nach Anspruch 5, ferner umfassend:
als Reaktion auf das Bestimmen, dass das Vertrauensniveau einen vorgegebenen Schwellenwert überschreitet, Speichern des geschätzten Sauerstoffsättigungsniveaus.

7. Verfahren nach Anspruch 5, ferner umfassend:
als Reaktion auf das Bestimmen, dass das Vertrauensniveau den vorgegebenen Schwellenwert nicht überschreitet, Verwerfen des geschätzten Sauerstoffsättigungsniveaus.

8. Verfahren nach einem der vorhergehenden Ansprüche, ferner umfassend:
Anzeigen des geschätzten Sauerstoffsättigungswerts.

9. Verfahren nach einem der vorhergehenden Ansprüche, wobei die elektrischen Signale einen Wechselstromanteil des Signals und einen Gleichstromanteil des Signals umfassen.

10. Verfahren nach einem der vorhergehenden Ansprüche, wobei die Lichtquellen LEDs sind.

11. Verfahren nach Anspruch 10, wobei die eine oder mehreren grünen LEDs Licht mit einer Wellenlänge zwischen 500 nm und 565 nm erzeugen, die eine oder mehreren roten LEDs Licht mit einer Wellenlänge zwischen 625 nm und 750 nm erzeugen und/oder die eine oder mehreren Infrarot-LEDs Licht mit einer Wellenlänge von mehr als 750 nm erzeugen.

12. Verfahren nach einem der vorhergehenden Ansprüche, wobei das Verfahren von einem tragbaren Rechengerät (100) durchgeführt wird.

13. Verfahren nach Anspruch 12, wobei das tragbare Rechengerät eine Smartwatch ist.

14. Tragbares Rechengerät (100), umfassend:

eine oder mehrere grüne Lichtquellen, eine oder mehrere rote Lichtquellen und eine oder mehrere infrarote Lichtquellen (202, 206, 910) ;
einen oder mehrere Lichtsensoren (204, 912);
einen oder mehrere Prozessoren (902); und
einen nichtflüchtigen computerlesbaren Speicher (904), wobei der Speicher Anweisungen speichert, die, wenn sie von dem einen oder den mehreren Prozessoren ausgeführt werden, das tragbare Rechengerät veranlassen, das Verfahren nach einem der Ansprüche 1 bis 11 und 13 durchzuführen.

15. Computerprogrammanweisungen, optional gespeichert auf einem computerlesbaren Medium, die, wenn sie von einer oder mehreren Rechengeräten einer Vorrichtung ausgeführt werden, die eine oder mehrere grüne Lichtquellen, eine oder mehrere rote Lichtquellen, eine oder mehrere Infrarotlichtquellen und einen oder mehrere Lichtsensoren umfasst, das eine oder die mehreren Rechengeräte veranlassen, das Verfahren nach einem der Ansprüche 1 bis 13 durchzuführen.

**Revendications**

1. Procédé pouvant être mis en œuvre par ordinateur pour mesurer la saturation en oxygène du sang chez un utilisateur, le procédé comprenant :

l'émission, à l'aide d'une ou plusieurs sources de lumière verte (202, 206, 910), de lumière vers un peau d'un utilisateur à une longueur d'onde verte ;
l'émission, à l'aide d'une ou plusieurs sources de lumière rouge (202, 910), de lumière vers une peau d'un utilisateur à une longueur d'onde rouge ;

l'émission, à l'aide d'une ou plusieurs sources de lumière infrarouge (202, 910), de lumière vers une peau d'un utilisateur à une longueur d'onde infrarouge ;

la détection, par un ou plusieurs capteurs de lumière (204, 912), d'une intensité de lumière verte, de lumière rouge, et de lumière infrarouge émise par la peau d'un utilisateur, dans lequel les un ou plusieurs capteurs de lumière génèrent, pour chacune de la lumière verte, de la lumière rouge et de la lumière infrarouge, un signal électrique respectif au fil du temps, formant une série temporelle de valeurs d'intensité pour chaque longueur d'onde, le signal électrique respectif à une longueur d'onde particulière représentant l'intensité de la longueur d'onde de la lumière, **caractérisé en ce que** le procédé comprend également :

le filtrage du signal électrique de la lumière rouge et du signal électrique de la lumière infrarouge à l'aide du signal électrique associé à la lumière verte par :

la génération d'un produit scalaire d'une série temporelle du signal électrique associé à la lumière verte et d'une série temporelle du signal électrique associé à la lumière rouge, et

la génération d'un produit scalaire de la série temporelle du signal électrique associé à la lumière verte et d'une série temporelle du signal électrique associé à la lumière infrarouge ; et

l'utilisation des signaux électriques filtrés pour déterminer un niveau de saturation en oxygène estimé.

2. Procédé selon la revendication 1, comprenant également :
la génération de données de caractéristiques à partir de l'intensité détectée de la lumière verte, de la lumière rouge et de la lumière infrarouge.

3. Procédé selon la revendication 2, dans lequel les données de caractéristiques comprennent des corrélations entre de la lumière rouge ou infrarouge et de la lumière verte et/ou le rapport entre une perfusion rouge et une perfusion verte.

4. Procédé selon la revendication 2 ou la revendication 3, comprenant également :
la détermination, à l'aide d'un modèle appris par machine avec les données de caractéristiques en entrée, d'un niveau de confiance associé au niveau de saturation en oxygène estimé du sang de l'utilisateur.

5. Procédé selon la revendication 4, comprenant également :
le fait de déterminer si le niveau de confiance dépasse un seuil prédéterminé.

6. Procédé selon la revendication 5, comprenant également :
en réponse à la détermination selon laquelle le niveau de confiance dépasse un seuil prédéterminé, le stockage du niveau de saturation en oxygène estimé.

7. Procédé selon la revendication 5, comprenant également :
en réponse à la détermination selon laquelle le niveau de confiance ne dépasse pas un seuil prédéterminé, la suppression du niveau de saturation en oxygène estimé.

8. Procédé selon une quelconque revendication précédente, comprenant également :
l'affichage du niveau de saturation en oxygène estimé.

9. Procédé selon une quelconque revendication précédente, dans lequel les signaux électriques comportent une partie de courant alternatif du signal et une partie de courant continu du signal.

10. Procédé selon une quelconque revendication précédente, dans lequel les sources de lumière sont des DEL.

11. Procédé selon la revendication 10, dans lequel les une ou plusieurs DEL vertes génèrent une lumière qui présente une longueur d'onde comprise entre 500 nm et 565 nm, les une ou plusieurs DEL rouges génèrent une lumière qui présente une longueur d'onde comprise entre 625 nm et 750 nm, et/ou les une ou plusieurs DEL infrarouges génèrent une lumière qui présente une longueur d'onde supérieure à 750 nm.

12. Procédé selon une quelconque revendication précédente, dans lequel le procédé est réalisé par un dispositif informatique portable (100).

13. Procédé selon la revendication 12, dans lequel le dispositif informatique portable est une montre intelligente.

14. Dispositif informatique portable (100), le dispositif informatique portable comprenant :

une ou plusieurs sources de lumière verte, une ou plusieurs sources de lumière rouge et une ou plusieurs sources de lumière infrarouge (202, 206, 910) ;

un ou plusieurs capteurs de lumière (204, 912) ;
un ou plusieurs processeurs (902) ; et
une mémoire lisible par ordinateur (904), dans lequel la mémoire lisible par ordinateur stocke des instructions qui, lorsqu'elles sont exécutées par les un ou plusieurs processeurs, amènent le dispositif informatique portable à réaliser le procédé selon l'une quelconque des revendications 1 à 11 et 13.

15. Instructions de programme informatique, éventuellement stockées sur un support lisible par ordinateur, qui, lorsqu'elles sont exécutées par un ou plusieurs dispositifs informatiques d'un dispositif comprenant une ou plusieurs sources de lumière verte, une ou plusieurs sources de lumière rouge, une ou plusieurs sources de lumière infrarouge et un ou plusieurs capteurs de lumière, amènent les un ou plusieurs dispositifs informatiques à réaliser le procédé selon l'une quelconque des revendications 1 à 13.

**FIG. 1**

100

204
206
202

FIG. 2

**FIG. 3**

FIG. 4

EP 4 351 412 B1

FIG. 5

EP 4 351 412 B1

FIG. 6

FIG. 7

EP 4 351 412 B1

FIG. 8

900

WEARABLE COMPUTING DEVICE — 900

PROCESSOR(S) — 902

MEMORY — 904

DATA — 906

INSTRUCTIONS — 908

LIGHT EMITTING DIODES — 910

LIGHT SENSOR — 912

SATURATION ESTIMATION SYSTEM — 914

FILTERING SYSTEM — 916

OXYGENATION DETERMINATION SYSTEM — 918

CONFIDENCE DETERMINATION SYSTEM — 920

DISPLAY SYSTEM — 922

# FIG. 9

EMIT, USING A GREEN LIGHT SOURCE, LIGHT TOWARDS A SKIN SOURCE OF A USER AT A GREEN WAVELENGTH — 1002

EMIT, USING A RED LIGHT SOURCE, LIGHT TOWARDS A SKIN SOURCE OF A USER AT A RED WAVELENGTH — 1004

EMIT, USING A INFRARED LIGHT SOURCE, LIGHT TOWARDS A SKIN SOURCE OF A USER AT A INFRARED WAVELENGTH — 1006

DETECT AN INTENSITY OF GREEN LIGHT, RED LIGHT, AND INFRARED LIGHT EMITTED FROM THE SKIN SURFACE OF A USER — 1008

GENERATE AN ESTIMATED OXYGEN SATURATION LEVEL — 1010

FIG. 10

# EP 4 351 412 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016361004 A
- US 10568525 B